# EUROPEAN PATENT SPECIFICATION

(11) **EP 0 328 634 B1**
(45) Date of publication and mention of the grant of the patent: **05.07.1995**
(21) Application number: 88908544.5
(22) Date of filing: 01.09.1988
(51) Int. Cl.: C07C 233/00, C07C 233/45

(54) **PHARMACEUTICAL COMPOSITIONS FOR THE TREATMENT OF PSORIASIS**
ARZNEIMITTELZUSAMMENSETZUNGEN ZUR BEHANDLUNG VON PSORIASIS
COMPOSES PHARMACEUTIQUES POUR LE TRAITEMENT DU PSORIASIS

(30) Priority: 04.09.1987 IL 83775
(43) Date of publication of application: 23.08.1989
(73) Proprietor: DEXTER CHEMICAL CORPORATION, Bronx, NY 10474 (US)
(72) Inventor: BLANK, Izhak, 31 999 Haifa (IL)
(74) Representative: Henkel, Feiler, Hänzel & Partner
(86) International application number: US8802941
(87) International publication number: WO8901930

(56) References cited:
- DE-A- 2 252 345
- DE-A- 2 703 964
- US-A- 4 362 737
- US-A- 4 433 154
- US-A- 4 730 048
- J. MED. CHEM. vol. 29, 1986, pages 1650-1653,American Chemical Society; P.S. PORTOGHESE et al.: "Synthesis and opioid antagonist potencies of naltrexamine bivalent ligands with conformationally restricted spacers"
- BIOCHEM J. vol. 201, 1982, pages 189-198, The Biochemical Society, A.J. BARRETT et al. "L- transepoxysuccinyl-leucylamido(4-guanidino) butane (E-64) and its analogues as inhibitors of cysteine proteinases including cathepsins B,H and L."
- CHEMICAL ABSTRACTS, vol. 107, no. 7 August 17, 1987, page 780, abstract 59468q, Ohio, US; & JP-A-62 48 656 (SHOWA DENKO K.K.)
- Z. HAUTKR., vol. 59, no. 10, 25th January 1984, pages 671-679, Grosse Verlag, Berlin, DE. W. RAAB: "Treatment of psoriasis with fumaric acid and fumarates"
- MICROBIOL IMMUNOL., vol. 28, no. 1. 1984, pages 85-97; T. AMAMAOTO et al: "Effect of E-64, thiol protease inhibitor on the secondary anti-SRBC response in vitro"

## Description

Psoriasis is one of the most widespread chronic diseases. It affects about two percent of the adult white population, the most severe symptoms being shown by patients in the age groups between twenty and fifty years old.

Psoriasis is characterized by a greatly accelerated rate of epidermal turnover. Instead of the normal period of 28 days from the time of cell division in the basal layers until the cell is shed from the stratum corneum, in psoriasis this takes only about four days.

The causes and mechanism of development of psoriasis are unknown, and for this reason a completely effective treatment for this ailment does not yet exist. A great number of approaches have been tried, from the very old, based on natural tars, to the more modern using steroids, sporalene, etc. Tars are messy to apply and have only a limited effect. Their combination with sulfur and salicylic acid are not much better. This therapy is frequently supplemented by the use of ultraviolet (UV) radiation, either natural (sunshine) or artificial (lamps). Other compounds used are: steroids, azaribine, methotrexate, psoralen, and retinoic acid derivatives. All of these have a rather high toxicity and their long term use may result in noxious side effects.

A possible approach to the therapy of the disease is to try to influence cellular metabolism, which obviously is much more active in the psoriatic cells than in the normal ones.

A few years ago, a new treatment was proposed. This is based on the use of fumaric acid in the form of its simple mono- or diesters or its metal salts, based on the theory that in the psoriatic portions of the skin there exists an unbalance in the dicarboxylic-acids cycle conducive to lower levels of fumarate. This theory seems to be confirmed by the fact that some amino acids, such as glycine, are present in lower quantities in the psoriatic skin, compared to their content in normal skin. Since these amino acids are also derived from the dicarboxylic-acids cycle, their presence in lower quantities is an added corroboration to the above theory.

A number of patent applications deal with the use of fumarate esters and salts for the treatment of psoriasis. DE-A-2530372 (13.1.77) describes the use of fumaric acid, fumarate esters, such as monoethyl and monomethyl fumarate, dimethyl fumarate; some salts of the monoesters such as manganese, calcium, zinc, iron, etc. All of these can be mixed with other ingredients such as tartaric acid, citric acid, sugar, and inert fillers. Some of these formulations are for internal use and some for external (topical) application. Related applications, DE-A-2840498 (10.4.80) and DE-A-2901452 (17.7.80), describe the addition of glycine, 1-methionine, and 1-cysteine to the above mixtures of fumarate esters and salts. A recent European patent, EP-A-01887419 (30.7.86) claims the use of fumarate esters of alcohols having one to eight carbon atoms, esters of higher alcohols (C6-C24), metal salts of the monoesters, and esters of diols, glycerol, and other hydroxyl-containing compounds. Another patent, DE-A-3232883 mentions the preparation of salts of fumaric acid with various caffein-8-ethers. The salts are crystalline and can be used for the preparation of tablets, capsules, etc., in combination with metal salts of fumaric esters, as mentioned before, and also with the optional addition of amino acids such as cysteine and methionine, and of vitamin C.

DE-A-2703964 discloses pharmaceutical compositions for the treatment of psoriasis comprising fumaric acid or derivatives thereof in combination with glycine and optional with sec. phosphates.

There exist serious problems as to the use of the above in the therapy of psoriasis. Short-chain fumarate esters are in general irritating materials which frequently produce an unpleasant acidosis effect upon ingestion. Metal salts of the half esters are quickly converted in the stomach into the free acid and the respective metal hydrochloride. The same happens with the caffein-ether salt. The esters are liquid at room temperature and in order to convert them to tablets they have to be adsorbed on, or mixed with, a rather large quantity of inert carrier. Furthermore, they have a strong characteristic odor and their toxicology has not been studied extensively. According to a study made with mice, monoethyl fumarate and dimethyl fumarate given per os had an LD₅₀ above 100 mg/kg. Monoethyl fumarate, given intraperitoneally, was more toxic (W. Raab, H&G Nr. 10 (1984)). These fumarate esters are highly irritating to the skin and can produce contact urticaria (Lahty et al., Contact Dermatitis 3, 139-140 (1985)).

To summarize: mono and diesters of fumaric acid have been shown to be effective in the treatment of psoriasis, as the experience with several thousand patients indicates (see, for instance: Schafer G. Fumarsauretherapie der Psoriasis, Arztliche Praxis 30, 61 p. 1757-58 (1978); also, Selecta 15, p. 1260-61 (1984)). The esters are irritating to the digestive system and to the skin and their toxicology has not been clearly established; they are also difficult to formulate as tablets.

Recent studies have shown that in psoriatic skin the content of glycine and serine is about twenty-five percent lower than in normal skin (Thaler et al., J. Invest. Dermatol. 75, 156-158 (1980); also, Steinert et al., Biochemistry of Normal and Abnormal Epidermal Differentiation, eds. I.A. Bereinstein and M. Seiji, Tokyo University Press, p. 391-406 (1980)). This deficiency may be related to the fumarate imbalance or to other unknown causes. The addition of glycine as such, to such formulations, cannot contribute much to the therapeutic effect since this water-soluble material will be quickly incorporated into the general metabolic processes, so, at best, its value will be like an added food.

### Brief Description of The Invention

We have found that linking amino acids such as glycine, serine, etc., to fumaric acid via a chemical link such as via amide groups, results in conjugates which have a high efficacy in the treatment of psoriasis. The conjugate compounds are mostly stable crystalline solids. They are easy to formulate as tablets, ointments, or similar galenic forms. The amide bond is known to be more stable to hydrolysis than an ester group (see, for instance, J.Marach, Advanced Organic Chemistry 3rd ed. p. 339, J. Wiley & Sons, New York (1985)), and therefore the fumar-amido amino acids are converted at a much slower rate into the fumarate and the free amino acid. They are easily absorbed through the digestive system, since it is known that amides have good solubilization properties both with hydrophilic and lipophilic compounds.

In its broadest aspects, therefore, the invention relates to compositions and methods for delivering a residue of fumaric acid and one or more amino acids to humans. It has been found that the compositions of the invention alleviate the symptoms of psoriasis. It has also been found that the compositions of the invention when administered per os have the effect of stimulating digestion and appetite, and when administered per os or topically reduce the tanning effects of the sun.

### Detailed Description of The Invention

The compounds of the invention include compounds of the formula 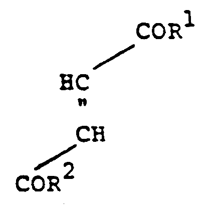
wherein R¹ and R², which are the same or different, each designates
(a) an ester of an amino acid, or
(b) an OH group, provided that only one of said R¹ and R² may be OH, where the amino acids are selected from one or more of: glycine, serine, valine, histidine, methionine, threonine, leucine, isoleucine, cysteine, cystine, tyrosine, proline, hydroxyproline, tryptophan, aspartic acid, glutamic acid, lysine, and arginine, as well as their derivatives, such as esters and salts
or a pharmaceutically acceptable salt thereof.
In the formulations it is possible to use the fumaramide of ethyl glycinate or of sodium glycinate. In other words, it is possible to make use of the carboxylic acid group of the amino acid to further change the solubility and other characteristics of the compound. Furthermore, since fumaric acid has two carboxyl groups, it is possible to prepare and make use of mixed amides, such as the glycine, serine fumaramide.

The amino acid esters of the compounds are desirably alkyl esters containing from 1 to 4 carbon atoms in the alkyl group.

The compositions of the invention may contain the active compounds described above, together with a pharmaceutically acceptable carrier as are known in the art. The carriers may include vehicles for immediate or sustained release and may be in a variety of dosage forms as are also known in the art.

The methods of the invention include, broadly, a method for delivering residues of fumaric acid and/or amino acids to a patient by administering the compositions of the invention, either per os or topically, as circumstances dictate. The compositions of the invention may be used to alleviate the symptoms of psoriasis. They may also be used to stimulate the appetite, and to reduce the tanning effects of the sun.

The materials of this invention are nonirritating to the skin, and preliminary toxicological studies with the diethyl ester of diglycyl fumaramide showed an LD50 above 5 g/kg (per os in rats). The amide conjugates are mild and nonirritating. Glycine is used in some formulations of aspirin tablets with the object of reducing gastric irritation. Any amount of glycine produced in the stomach by hydrolysis of the amide, will actually act in a beneficial way, in this respect.

The invention is illustrated by the following example.

All quantities are given in parts by weight.

### Example : = Ethyl ester of diglycyl fumaramide (EGFA)

Glycine ether ester hydrochloride 13.9 parts, fumaryl chloride 7.65 parts, and 8.8 parts of sodium hydroxide in water were reacted as above. After purification the material obtained is an off-white powder. N(calc.)9.7; found: 10.1.

## Claims

1. A compound of the formula: 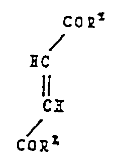 wherein R¹ and R², which are the same or different, each designates
(a) an ester of an amino acid, or
(b) an OH group, provided that only one of such R¹ and R² may be OH,
where the amino acids are selected from glycine, serine, valine, histidine, threonine, leucine, isoleucine, cysteine, cystine, methionine, tyrosine, proline, hydroxyproline, tryptophan, aspartic acid, glutamic acid, lysine, and arginine, or a pharmaceutically acceptable salt thereof.

2. A compound as recited in claim 1, where the amino acids residue R¹ and R² is linked to the fumaric acid residue by an amido linkage.

3. A compound as recited in claim 1, where the amino acids are selected from glycine and serine.

4. A compound as recited in claim 1, where the amino acid esters are C₁- to C₄-alkyl esters.

5. A compound selected from the C₁- to C₄-alkyl esters of di-glycine-fumaramide, glycine-serine-fumaramide, di-serine-fumaramide and glycine-lauryl-fumaramide.

6. A compound selected from group consisting of the C₁- to C₄-alkyl esters of glycine-serine-fumaramide, di-serine-fumaramide and glycine-lauryl-fumaramide.

7. The diethyl ester of diglycyl fumaramide.

8. A pharmaceutical composition which contains an active compound of the formula: 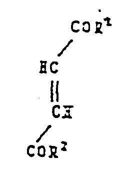 or a pharmaceutically acceptable salt thereof, wherein R¹ and R², which are the same or different, each designates
(a) an ester of an amino acid, or
(b) an OH group, provided that only one of such R¹ and R² may be OH,
where the amino acids are selected from glycine, serine, valine, histidine, threonine, leucine, isoleucine, cysteine, cystine, methionine, phenylalanine, tyrosine, proline, hydroxyproline, tryptophan, aspartic acid, glutamic acid, lysine, and arginine; and a pharmaceutically acceptable carrier.

9. A composition as recited in claim 8, for administration per os.

10. A composition as recited in claim 8, for topical application.

11. A composition as recited in claim 8, 9, or 10, where the active ingredient is selected from the group consisting of esters of monoglycyl fumaramide, diglycyl fumaramide, mono-serine fumaramide, and di-serine fumaramide.

12. A pharmaceutical composition according to claims 8 - 11 for alleviating the symptoms of psoriasis and other skin diseases.

13. A pharmaceutical composition according to claim 8 for stimulating digestion or appetite.

14. A pharmaceutical composition according to claim 8 for reducing the tanning effect of exposure to the sun.

15. A pharmaceutical composition as claimed in claim 10 for delivering the residue of fumaric acid and/or one or more amino acids to humans.

16. Use of a composition as claimed in claim 10 for the manufacture of a medicament for the treatment of psoriasis.

17. Use of a composition as claimed in claim 13 for the manufacture of a medicament for stimulating digestion or appetite in humans.

18. Use of a composition as claimed in claim 14 for reducing the tanning effect of exposure to the sun.

## Patentansprüche

1. Verbindung der Formel: 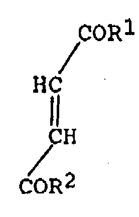 worin R¹ und R², die gleich oder verschieden sind, jeweils
(a) einen Ester einer Aminosäure oder
(b) eine OH-Gruppe bedeuten, wobei gilt, daß lediglich einer der Reste R¹ und R² für eine OH-Gruppe steht und wobei die Aminosäuren aus Glycin, Serin, Valin, Histidin, Threonin, Leucin, Isoleucin, Cystein, Cystin, Methionin, Tyrosin, Prolin, Hydroxyprolin, Tryptophan, Asparaginsäure, Glutaminsäure, Lysin und Arginin ausgewählt sind,
oder ein pharmazeutisch akzeptables Salz derselben.

2. Verbindung nach Anspruch 1, wobei die Aminosäurereste R¹ und R² über eine Amidbindung an den Fumarsäurerest gebunden sind.

3. Verbindung nach Anspruch 1, wobei die Aminosäuren aus Glycin und Serin ausgewählt sind.

4. Verbindung nach Anspruch 1, wobei es sich bei den Aminosäureestern um C₁-C₄-Alkylester handelt.

5. Verbindung, ausgewählt aus den C₁-C₄-Alkylestern von Di-glycin-fumaramid, Glycin-serin-fumaramid, Di-serin-fumaramid und Glycin-lauryl-fumaramid.

6. Verbindung, ausgewählt aus der Gruppe C₁-C₄-Alkylester von Glycin-serin-fumaramid, Di-serin-fumaramid und Glycin-lauryl-fumaramid.

7. Der Diethylester von Di-glycyl-fumaramid.

8. Arzneimittelzubereitung, enthaltend eine aktive Verbindung der Formel: 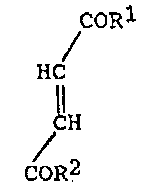 oder ein pharmazeutisch akzeptables Salz derselben, wobei R¹ und R², die gleich oder verschieden sind, jeweils
(a) einen Ester einer Aminosäure oder
(b) eine OH-Gruppe bedeuten, wobei gilt, daß lediglich einer der Reste R¹ und R² für eine OH-Gruppe steht und wobei die Aminosäuren aus Glycin, Serin, Valin, Histidin, Threonin, Leucin, Isoleucin, Cystein, Cystin, Methionin, Phenylalanin, Tyrosin, Prolin, Hydroxyprolin, Tryptophan, Asparaginsäure, Glutaminsäure, Lysin und Arginin ausgewählt sind,
sowie einen pharmazeutisch akzeptablen Träger.

9. Zubereitung nach Anspruch 8 zur Verabreichung per os.

10. Zubereitung nach Anspruch 8 zur topischen Applikation.

11. Zubereitung nach Anspruch 8, 9 oder 10, wobei der aktive Bestandteil aus der Gruppe Ester von Mono-glycyl-fumaramid, Di-glycyl-fumaramid, Mono-serin-fumaramid und Di-serin-fumaramid ausgewählt ist.

12. Arzneimittelzubereitung nach Ansprüchen 8 bis 11 zur Linderung der Symptome von Psoriasis und anderer Hautkrankheiten.

13. Arzneimittelzubereitung nach Anspruch 8 zur Anregung der Verdauung oder des Appetits.

14. Arzneimittelzubereitung nach Anspruch 8 zur Verminderung des Bräunungseffekts bei Sonneneinwirkung.

15. Arzneimittelzubereitung nach Anspruch 10 zur Abgabe der Reste von Fumarsäure und/oder einer oder mehrerer Aminosäure(n) an Menschen.

16. Verwendung einer Zubereitung nach Anspruch 10 zur Herstellung eines Medikaments zur Behandlung von Psoriasis.

17. Verwendung einer Zubereitung nach Anspruch 13 zur Herstellung eines Medikaments zur Anregung der Verdauung oder des Appetits beim Menschen.

18. Verwendung einer Zubereitung nach Anspruch 14 zur Verminderung des Bräunungseffekts bei Sonneneinwirkung.

## Revendications

1. Composé de formule : 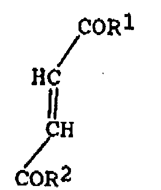 dans laquelle R¹ et R², qui sont les mêmes ou qui sont différents, désignent chacun
(a) un ester d'un acide amino, ou
(b) un groupe OH, avec la condition que seulement l'un de R¹ et R² puisse être OH,
dans lequel les acides amino sont sélectionnés parmi la glycine, la sérine, la valine, l'histidine, la thréonine, la leucine, l'isoleucine, la cystéine, la cystine, la méthionine, la tyrosine, la proline, l'hydroxyproline, le tryptophan, l'acide aspartique, l'acide glutamique, la lysine et lʼarginine, ou l'un de leurs sels pharmaceutiquement acceptables;

2. Un composé selon la revendication 1, dans lequel le, résidu l'acides amino R¹ et R² est lié au résidu d'acide fumarique par une liaison amido.

3. Un composé selon la revendication 1, dans lequel les acides amino sont sélectionnés parmi la glycine et la sérine.

4. Un composé selon la revendication 1, dans lequel les esters d'acide amino sont des esters C₁- à C₄-alkyle.

5. Un composé sélectionné parmi les esters C₁- à C₄-alkyle, de di-glycine-fumaramide, de glycine-sérine-fumaramide, de di-sérine-fumaramide et de glycine-lauryle-fumaramide.

6. Un composé sélectionné dans le groupe constitué par les esters C₁- à C₄-alkyle de glycine-sérine-fumaramide, de di-sérine-fumaramide et de glycine-lauryle-fumaramide.

7. Le diéthyle ester de diglycyle fumaramide.

8. Une composition pharmaceutique qui contient un composé actif de formule : 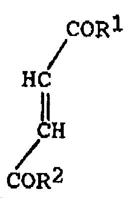 ou l'un de ses sels pharmaceutiquement acceptables, dans laquelle R¹ et R², qui sont les mêmes ou différents, désignent chacun
(a) un ester d'un acide amino, ou
(b) un groupe OH, avec la condition que seulement l'un de R¹ et R² puisse être OH,
dans lequel les acides amino sont sélectionnés parmi la glycine, la sérine, la valine, l'histidine, la thréonine, la leucine, l'isoleucine, la cystéine, la cystine, la méthionine, la phénylalanine, la tyrosine, la proline, l'hydroxyproline, le tryptophan, l'acide aspartique, l'acide glutamique, la lysine et l'arginine ; et un support pharmaceutiquement acceptable.

9. Une composition selon la revendication 8, pour l'administration per os.

10. Une composition selon la revendication 8, pour l'administration topique.

11. Une composition selon la revendication 8, 9, ou 10, dans laquelle l'ingrédient actif est sélectionné dans le groupe constitué par les esters de monoglycyle fumaramide, de diglycyle fumaramide, de mono-sérine fumaramide et de diserine fumaramide.

12. Une composition pharmaceutique selon les revendications 8 - 11 pour atténuer les systômes du psoriasis et autres maladies de la peau.

13. Une composition pharmaceutique selon la revendication 8 pour stimuler la digestion ou l'appétit.

14. Une composition pharmaceutique selon la revendication 8 pour réduire l'effet de hâle de l'exposition au soleil.

15. Une composition pharmaceutique telle que revendiquée dans la revendication 10 pour délivrer à l'homme le résidu d'acide fumarique et/ou d'un ou plus d'un acide amino.

16. Utilisation d'une composition telle que revendiquée dans la revendication 10 pour la fabrication d'un médicament pour le traitement du psioriasis.

17. Utilisation d'une composition telle que revendiquée dans la revendication 13 pour la fabrication d'un médicament pour stimuler la digestion ou l'appétit chez l'homme.

18. Utilisation d'une composition telle que revendiquée dans la revendication 14 pour réduire l'effet de hâle de l'exposition au soleil.
